# EUROPEAN PATENT APPLICATION

(11) **EP 4 656 765 A1**
(43) Date of publication of application: **03.12.2025**
(21) Application number: 24178830.6
(22) Date of filing: 29.05.2024
(51) Int. Cl.: C23C 14/00, C23C 14/02, C23C 14/06, C23C 14/34, C23C 14/35, A61L 27/30, C23C 28/04

(54) **PROCESS FOR COATING AN IMPLANT AND AN IMPLANT HAVING A CERAMIC MULTI-LAYER COATING**

(71) Applicant: Aesculap AG, 78532 Tuttlingen (DE); IHI Ionbond AG, 4657 Dulliken (CH)
(72) Inventor: DOHM, Julius, 61476 Kronberg (DE); RICHTER, Berna, 78570 München (DE); GRUPP, Thomas, 78588 Denkingen (DE); SCHMIDT, Susann, 4665 Oftringen (DE); SANTANA, Antonio, 2500-224 Caldas da Rainha (PT)
(74) Representative: Patentanwälte Ruff, Wilhelm, Beier, Dauster & Partner mbB

(57) **Abstract**

The invention relates to a process for coating an implant comprising the step of
- depositing a ceramic multi-layer coating on a surface of the implant via pulsed magnetron sputtering.

Further, the invention refers to an implant having a ceramic multi-layer coating.

## Description

### Field of the Invention

The present invention relates to a process for coating an implant and an implant having a ceramic multi-layer coating.

### Background of the invention

Application of hard, wear-resistant was well as oxidation-reducing multi-layer coatings on implants, in particular implants having concave geometries, is a challenging process. Such coatings typically require extensive post coat polishing steps to comply with required surface finishes, in particular to comply with a coating thickness of < 50 nm.

It is known to use a cathodic arc deposition process to apply multi-layer coatings. However, this process often results in coated surfaces having defects such as pin holes or droplets. Extensive post polishing is required for such surfaces, which is inherently difficult especially for concave geometries if certain standards, in particular of the Food and Drug Administration (FDA), have to be considered.

### Object and Solution

In view of the foregoing, the object underlying the present invention is therefore to make available a process to coat an implant which circumvents or at least mitigates detriments as described above. Further, it is an object of the present invention to provide an implant having a ceramic multi-layer coating.

This object is accomplished by a process according to independent claim 1 and by an implant according to independent claim 13. Preferred embodiments are defined in the dependent claims and the present description. The subject-matter and wording, respectively, of all claims is hereby incorporated into the description by explicit reference.

According to a first aspect, the invention relates to a process for coating an implant, in particular medical or surgical implant, comprising the step of
- depositing or applying a ceramic multi-layer coating on a surface of the implant via, i.e. by the aid of, pulsed magnetron sputtering.

The term "ceramic multi-layer coating" as used according to the present invention refers to a multi-layer coating, i.e. a coating comprising or consisting of a multitude of layers, i.e. two or more layers, preferably two, three, four, five, six, seven or eight layers, more preferably seven or eight layers, wherein at least some of the layers, preferably all layers, of the coating comprise or consist of a ceramic material, in particular hard ceramic material, in particular as detailed in the following description.

The invention inter alia rests on the surprising finding that by depositing a ceramic multi-layer coating on an implant's surface, the detriments known from the prior art can be properly addressed. Particularly, it turned out that application of pulsed magnetron sputtering resulted in less droplets, pin holes and porosity compared to standard cathodic arc deposition, and therefore in a smoother surface showing a higher wear resistance and a lower friction coefficient, which is especially advantageous with respect to implants having a gliding or articulating surface. Thus, surface and coating defects may be advantageously reduced as well as the need for extensive post coat polishing. Further advantageously, the service life of the implant can thus be extended.

Compared to arc evaporation, pulsed magnetron sputtering, in particular pulsed DC magnetron sputtering and high-power pulse magnetron sputtering, utilize comparatively high cathode voltages to accelerate inert and/or reactive gas ions to a sputter target leading to the emission of target material (ions, atoms etc.). Arc evaporation, on the other hand, uses high target currents to melt small amounts of target material which is subsequently evaporated. Both deposition techniques utilize commercially available physical vapor evaporation (PVD) coating systems, built from stainless steel vacuum chambers and equipped with heaters, cooling water systems, pumping systems allowing for base pressures < 5 mPa, rotating substrate tables connected to a bias power supply and cathodes on which pure, target materials, in particular metal targets, are mounted. In contrast to arc evaporation, target and/or bias voltages for pulsed magnetron sputtering are preferably supplied at frequencies between 10 Hz to 900 MHz and are thus suitable techniques for poorly conductive coatings. For further details of the pulsed DC magnetron sputtering and high-power impulse magnetron sputtering, reference is made to the following description.

Pulsed magnetron sputtering largely utilizes the basic principles of magnetron sputter deposition. Magnetron sputtering is a relatively violent, atomic-scale process generally carried out in diode plasma systems known as magnetrons. On each magnetron, a target source, for example a pure metal, is bombarded with ions of a sputtering gas, for example argon and/or nitrogen. When struck by the gas ions, target atoms and/or ions are emitted and guided towards the substrate to produce a thin film composed of the sputtered target species.

To carry out magnetron sputtering, a permanent magnet structure may be located behind the target serving as a deposition source. Plasma confinement on the target surface may also be achieved by locating a permanent magnet structure behind the target surface. The magnets are used to confine electrons in the plasma, resulting in higher plasma densities and consequently reducing the discharge impedance and results in a much higher current, lower-voltage discharge. The resulting magnetic field forms a closed-loop annular path acting as an electron trap that reshapes the trajectories of secondary electrons ejected from the target into a cycloidal path, greatly increasing the probability of ionization of the sputtering gas within the confinement zone. Inert and/or reactive gases, such as argon and/or nitrogen, are used as the sputtering gas to achieve the desired coating composition i.e. metallic in case only argon is used or a mix of nitrogen and argon for a stoichiometric ceramic coating. Inert gases such as argon do not react with the target material or combine with reactive process gases but provide ions to increase higher sputter yields and thus deposition rates. To deposit a metal nitride such as a metal oxynitride as a ceramic material, nitrogen is used to supply the nitrogen ions. Here, nitrogen is ionized mainly by electron impact in the gas phase, the nitrogen then reacts with the metal target or metal particles in the gas phase and a ceramic film is deposited on the substrates.

Typically, pulsed magnetron sputtering is carried out using a deposition chamber as described above. Additionally, the deposition chamber comprises at least one pulsed power supply connected to at least one magnetron (cathode).

Principally, the pulsed magnetron sputtering, according to the present invention, may be carried out as a pulsed DC (direct current) magnetron sputtering, a RF (radio frequency) magnetron sputtering, a MF (mid-frequency) magnetron sputtering, a high-power impulse magnetron sputtering or a combination thereof.

In an embodiment of the invention, the pulsed magnetron sputtering is carried out as a pulsed DC (direct current) magnetron sputtering. Pulsed DC (direct current) magnetron sputtering is based on the addition of a reverse-voltage pulse to the normal DC waveform. This pulse, in particular provided by a pulse controller, when implemented at a frequency high enough to exploit the mobility differences between the ions and electrons in the plasma, accentuates the sputtering of films, in particular dielectric films, that accumulate on a target surface and effectively eliminates target poisoning and arcing. In particular, each magnetron target acts alternatively as an anode and a cathode during the pulse cycle providing very long-term process stability at enhanced deposition rates. The magnetron may operate in an asymmetric bipolar mode at the repetition frequency of pulses in the range from, for example, 10 kHz to 300 kHz. The sputtering takes place from the target during a negative voltage pulsed, while discharging of the target surface takes place during a successive positive voltage pulse (typically 10 % of the nominal "on" voltage).

In a further embodiment of the invention, the pulsed magnetron sputtering is carried out as high-power impulse magnetron sputtering. High-power impulse magnetron sputtering (HIPIMS or HiPIMS), also known as high-power pulsed magnetron sputtering (HPPMS), is a process for physical vapor deposition of thin films which is based on magnetron sputter deposition. HIPIMS utilizes extremely high power densities, in particular of the order of kW cm⁻² in short pulses (impulses) of tens of microseconds at much lower frequencies compared to DC magnetron sputtering, in particular in the order of 10 Hz to 10 kHz, in particular in the order of 10 Hz to < 10 kHz, and low duty cycle (on/off time ratio) of < 10%. Distinguishing features of HIPIMS are a high degree of ionization of the sputtered material and a high rate of molecular gas dissociation which results in high density of deposited films. The degree of ionization and dissociation increases according to the peak cathode power. The limit is determined by the transition of the discharge from glow to arc phase. Typically, the peak power and the duty cycle are selected so as to maintain an average cathode power similar to conventional sputtering (1-10 W cm⁻²).

A HIPIMS plasma is generated by a glow discharge where the discharge current density can reach several A cm⁻², while the discharge voltage is maintained at several hundred volts. The discharge is homogeneously distributed across the surface of the cathode (target), however, above a certain threshold of current density it becomes concentrated in narrow ionization zones that move along a path known as the target erosion "racetrack".

HIPIMS generates a high-density plasma, in particular of the order of 10¹³ ions cm⁻³ containing high fractions of target material ions. The main ionization mechanism is electron impact, which is balanced by charge exchange, diffusion, and plasma ejection in flares. The ionization rates depend on the plasma density. The ionization degree of the target vapor is a strong function of the peak current density of the discharge. At high current densities, sputtered ions with a charge 2+ and higher - up to 5+ for Vanadium - can be generated. The appearance of target ions with charge states higher than 1+ is responsible for a potential secondary electron emission process that has a higher emission coefficient than the kinetic secondary emission found in conventional glow discharges. The establishment of a potential secondary electron emission may enhance the current of the discharge. HIPIMS is typically operated in short pulse (impulse) mode with a low duty cycle to avoid overheating of the target and other system components. In every pulse the discharge goes through several stages: electrical breakdown, gas plasma, target material plasma and steady state, which may be reached if the target material plasma is dense enough to effectively dominate over the gas plasma.

The on-off cycle has preferably a period in the order of milliseconds. Because the duty cycle is low (preferably < 10%), a comparatively low average cathode power can be maintained (preferably 1-20 kW). The target can cool down during the "off time", thereby maintaining process stability.

The discharge that maintains HIPIMS is a comparatively high-current glow discharge, which is transient or quasi-stationary. Each pulse remains a glow up to a critical duration after which it transits to an arc discharge. If the pulse length is kept below the critical duration, the discharge operates in a stable fashion indefinitely.

In a further embodiment of the invention, pulses having a peak power density of 0.01 kW/cm² to 30 kW/cm², in particular 0.01 kW/cm² to 0.5 kW/cm², preferably 0.01 kW/cm² to 0.1 kW/cm², or pulses having a peak power density of 0.1 kW/cm² to 30 kW/cm², in particular 0.1 kW/cm² to 10 kW/cm², preferably 0.1 kW/cm² to 5 kW/cm², are generated during the pulsed magnetron sputtering. Preferably, pulses having a peak power density of 0.1 kW/cm² to 30 kW/cm², in particular 0.1 kW/cm² to 10 kW/cm², preferably 0.1 kW/cm² to 5 kW/cm², are generated, if the pulsed magnetron sputtering is carried out as high-power impulse magnetron sputtering.

Further, pulses having a peak current density of 0.01 A/cm² to 3 A/cm², in particular 0.5 A/cm² to 1 A/cm², are generated during the pulsed magnetron sputtering.

In a further embodiment of the invention, pulses having a frequency of 10 Hz to 300 kHz, in particular 20 Hz to 300 kHz, in particular 10 kHz to 300 kHz, preferably 10 kHz to 100 kHz, or pulses having a frequency of 10 Hz to 10 kHz or 10 Hz to < 10 kHz, in particular 20 Hz to 10 kHz or 20 Hz to < 10 kHz, preferably 40 Hz to 1000 Hz, are generated during the pulsed magnetron sputtering. Preferably, pulses having a frequency of 10 Hz to 10 kHz or 10 Hz to < 10 kHz, in particular 20 Hz to 10 kHz or 20 Hz to < 10 kHz, preferably 40 Hz to 1000 Hz, are generated, if the pulsed magnetron sputtering is carried out as high-power impulse magnetron sputtering.

In a further embodiment of the invention, pulses having a voltage of 200 V to 800 V, preferably 300 V to 700 V, are generated during the pulsed magnetron sputtering.

In a further embodiment of the invention, pulses having a duration of 10 µs to 200 µs, preferably 40 µs to 150 µs, are generated during the pulsed magnetron sputtering, preferably high-power impulse magnetron sputtering.

Further, the ceramic multi-layer coating may be deposited on the surface of the implant at a temperature of 120 °C to 400 °C, preferably 250 °C to 400 °C.

Further, the ceramic multi-layer coating may be deposited on the surface of the implant under a pressure of 10⁻³ mbar to 10⁻² mbar, preferably 2 × 10⁻³ mbar to 7 × 10⁻³ mbar.

In a further embodiment of the invention, a ceramic material selected from the group consisting of chromium nitride, chromium carbonitride, zirconium chromium nitride, zirconium nitride and combinations of at least two of the aforesaid ceramic materials is sputtered and deposited in layers on the surface of the implant during the pulsed magnetron sputtering to generate the ceramic multi-layer coating. The aforementioned ceramic materials have in particular the advantage that they are biocompatible materials.

In a further embodiment of the invention, further a material, in particular a metallic material, preferably zirconium, or a metal-containing material, in particular metal nitride, preferably zirconium nitride, or metal oxide, in particular zirconium oxide, is sputtered in the presence of oxygen, in particular molecular oxygen (dioxygen, O₂) and/or nitrogen, in particular molecular nitrogen (dinitrogen, N₂), during the pulsed magnetron sputtering to form an oxide layer or oxynitride layer, in particular a metal oxide layer or metal oxynitride layer, preferably an outer oxide layer or an outer oxynitride layer, in particular an outer metal oxide layer or an outer metal oxynitride layer, of the ceramic multi-layer coating. Preferably, the metal oxide layer is a zirconium oxide layer, in particular an outer zirconium oxide layer, of the ceramic multi-layer coating. The metal oxynitride layer is preferably a zirconium oxynitride layer, in particular an outer zirconium oxynitride layer, of the ceramic multi-layer coating. The oxide layer may have an oxide gradient or a discrete oxygen content.

The addition of an oxide layer or oxynitride layer, in particular a metal oxide layer or metal oxynitride layer, as an outer layer of the ceramic multi-layer coating advantageously stabilizes the oxidation rate of the ceramic multi-layer coating. Further advantageously, the addition of an oxide layer or oxynitride layer does not impair any of the properties of the ceramic multi-layer coating but has beneficial aspects such as reduced discoloration due to oxidation of an outer layer of the ceramic multi-layer coating and/or reduced wear of the ceramic multi-layer coating.

The term "oxide layer" as used according to the present invention refers to a layer comprising or consisting of an oxide.

The term "oxynitride layer" as used according to the present invention refers to a layer comprising or consisting of an oxynitride.

The term "metal oxide layer" as used according to the present invention refers to a layer comprising or consisting of a metal oxide.

The term "metal oxynitride layer" as used according to the present invention refers to a layer comprising or consisting of a metal oxynitride.

The term "zirconium oxide layer" as used according to the present invention refers to a layer comprising or consisting of zirconium oxide.

The term "zirconium oxynitride layer" as used according to the present invention refers to a layer comprising or consisting of zirconium oxynitride.

The term "outer oxide layer" as used according to the present invention refers to an oxide layer of the ceramic multi-layer coating that separates the ceramic multi-layer coating from its surroundings.

The term "outer oxynitride layer" as used according to the present invention refers to an oxynitride layer of the ceramic multi-layer coating that separates the ceramic multi-layer coating from its surroundings.

The term "outer metal oxide layer" as used according to the present invention refers to a metal oxide layer of the ceramic multi-layer coating that separates the ceramic multi-layer coating from its surroundings. Accordingly, the term "outer zirconium oxide layer" as used according to the present invention refers to a zirconium oxide layer of the ceramic multi-layer coating that separates the ceramic multi-layer coating from its surroundings.

The term "outer metal oxynitride layer" as used according to the present invention refers to a metal oxynitride layer of the ceramic multi-layer coating that separates the ceramic multi-layer coating from its surroundings. Accordingly, the term "outer zirconium oxynitride layer" as used according to the present invention refers to a zirconium oxynitride layer of the ceramic multi-layer coating that separates the ceramic multi-layer coating from its surroundings.

By applying an outer metal oxide layer or metal oxynitride layer, the oxidation rate of the implant's surface can be advantageously stabilized. Thus, the service life of the implant can be additionally extended. Furthermore, release of metal ions, and thus the friction coefficient of the implant's surface, can be (additionally) reduced.

In a further embodiment of the invention, the following layers are deposited, in particular directly or indirectly, preferably directly, on top of each other, on the surface of the implant, preferably in the order a) to g), during the pulsed magnetron sputtering to generate the ceramic multi-layer coating:
a) a chromium nitride layer,
b) a chromium carbonitride layer,
c) a chromium nitride layer,
d) a chromium carbonitride layer,
e) a chromium nitride layer,
f) a zirconium chromium nitride layer and
g) a zirconium nitride layer.

The term "chromium nitride layer" as used according to the present invention refers to a layer comprising or consisting of chromium nitride.

The term "chromium carbonitride layer" as used according to the present invention refers to a layer comprising or consisting of chromium carbonitride.

The term "zirconium chromium nitride" layer as used according to the present invention refers to a layer comprising or consisting of zirconium chromium nitride.

The term "zirconium nitride layer" as used according to the present invention refers to a layer comprising or consisting of zirconium nitride layer.

In a further embodiment of the invention, the following layers are deposited, in particular directly or indirectly, preferably directly, on top of each other, on the surface of the implant, preferably in the order a) to h), during the pulsed magnetron sputtering to generate the ceramic multi-layer coating:
a) a chromium nitride layer,
b) a chromium carbonitride layer,
c) a chromium nitride layer,
d) a chromium carbonitride layer,
e) a chromium nitride layer,
f) a zirconium chromium nitride layer,
g) a zirconium nitride layer and
h) an oxide layer or oxynitride layer, in particular a metal oxide layer, preferably zirconium oxide layer, or metal oxynitride layer, preferably zirconium oxynitride layer.

The advantages of the present invention are particularly evident in the two preceding embodiments.

In a further embodiment of the invention, the surface of the implant is or comprises a concave surface. Alternatively or in combination, the surface of the implant is or comprises a convex surface and/or an even surface.

In a further embodiment of the invention, the surface of the implant is or comprises an articulating or a gliding surface, in particular a surface of an artificial articular socket such as an artificial acetabula cup, an artificial acetabula liner, a femoral component or a shoulder prosthesis. Alternatively, the surface of the implant may be an inner surface of a head-bore.

In a further embodiment of the invention, the implant is a joint implant (articular implant), in particular selected from the group consisting of tibial implant, femoral implant, knee joint implant, hip joint implant, ankle joint implant, shoulder joint implant, mandibular joint implant, elbow joint implant, finger joint implant and spinal joint implant, or a component thereof. Particularly, the component may be in the form of an artificial articular socket. More preferably, the component may be a component of a hip joint implant, in particular an artificial acetabula cup or artificial acetabula liner.

According to a second aspect, the invention refers to an implant having a ceramic multi-layer coating. The implant is produced or producible according to a process according to the first aspect of the invention and/or the ceramic multi-layer coating comprises an oxide layer or oxynitride layer, in particular a metal oxide layer or metal oxynitride layer, as an outer layer.

Preferably, the metal oxide layer is a zirconium oxide layer.

Especially preferably, the metal oxynitride layer is a zirconium oxynitride layer, in particular having the formula ZrO_{y}Nₓ, wherein 1-x = y and x<1 apply.

In a further embodiment of the invention, the oxide layer or oxynitride layer, in particular metal oxide layer or metal oxynitride layer, in particular directly or indirectly, preferably directly, layers a zirconium nitride layer of the ceramic multi-layer coating.

In a further embodiment of the invention, the ceramic multi-layer coating comprises or consists of the following layers, preferably in the order a) to h):
a) a chromium nitride layer,
b) a chromium carbonitride layer,
c) a chromium nitride layer,
d) a chromium carbonitride layer,
e) a chromium nitride layer,
f) a zirconium chromium nitride layer,
g) a zirconium nitride layer and
h) the oxide layer or oxynitride layer, in particular metal oxide layer, preferably zirconium oxide layer, or metal oxynitride layer, preferably zirconium oxynitride layer.

Principally, the chromium nitride layer a) may directly or indirectly layer a surface of the implant. Preferably, the chromium nitride layer a) directly layers a surface of the implant.

Further, the layers a) to h) may be arranged directly or indirectly, preferably directly, on top of each other.

Further preferably, the ceramic multi-layer coating has a thickness, in particular measured according to ISO 26423 and/or ISO 1463, of 2 µm to 10 µm, preferably 3.5 µm to 6 µm.

Further preferably, the ceramic multi-layer coating has an average surface roughness, in particular measured according to ISO 21920-2 and/or ISO 21920-3, of < 0.04 µm, in particular 0.01 µm to 0.03 µm.

Further preferably, the ceramic multi-layer coating has a HRC (Hardness Rockwell C) adhesion, in particular measured according to ISO 26443, of ISO class 1 or better.

With respect to further features and advantages of the implant according to the second aspect of the present invention, reference is made in its entirety to the features and advantages mentioned under the first aspect of the present invention which do apply accordingly.

Further features and advantages of the invention will become clear from the following examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### Description of the figures

For a better understanding of what has been disclosed some figures are attached which solely by way of non-limiting example show a practical case of embodiment of the present invention.

In the figures, the following is displayed:

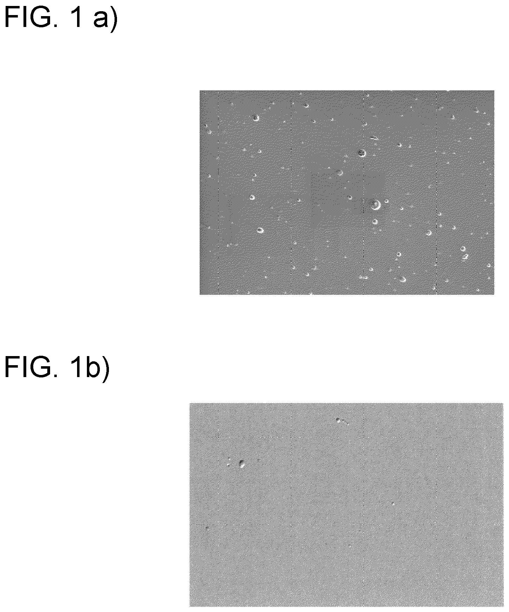

Fig. 1a shows a detailed SEM image of a surface of an applied zirconium nitride multi-layer coating via a PVD (physical vapor deposition) process and Fig. 1b shows a detailed SEM image of a surface of an applied zirconium nitride multi-layer coating via high-power impulse magnetron sputtering. From this it becomes clear that the application of pulsed magnetron sputtering produces a smoother surface with less defects visible.

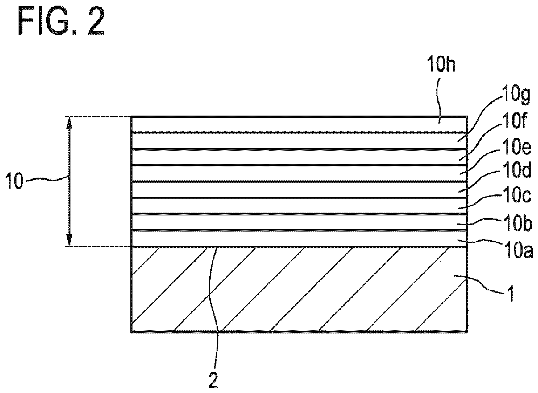

Fig. 2 schematically shows an enlarged detail representation of an implant 1 being coated with a ceramic multi-layer coating 10.

The ceramic multi-layer coating 10 has been deposited on a surface 2 of the implant 1 via pulsed magnetron sputtering, in particular pulsed DC (direct current) magnetron sputtering or high-power impulse magnetron sputtering.

The ceramic multi-layer coating 10 comprises or consists of eight layers 10a-h. The surface 2 of the implant 2 is directly coated by a first chromium nitride layer 10a. The first chromium nitride layer 10a is coated directly by a first chromium carbonitride layer 10b. The first chromium carbonitride layer 10b is coated directly by a second chromium nitride layer 10c. The second chromium nitride layer 10c is coated directly by a second chromium carbonitride layer 10d. The second chromium carbonitride layer 10d is coated directly by a third chromium nitride layer 10e. The third chromium nitride layer 10e is coated directly by a chromium zirconium nitride layer 10f. The chromium zirconium nitride layer 10f is coated directly by a zirconium nitride layer 10g. The zirconium nitride layer 10g is coated directly by a zirconium oxynitride layer 10h.

For further features and advantages in respect of the implant 1, the ceramic multi-layer coating 10 and the pulsed magnetron sputtering, in particular pulsed DC magnetron sputtering or high-power impulse magnetron sputtering, reference is made in its entirety to the features and advantages mentioned in the general description which do apply accordingly.

## Claims

1. A process for coating an implant comprising the step of
- depositing a ceramic multi-layer coating on a surface of the implant via pulsed magnetron sputtering.

2. The process according to claim 1, **characterized in that** the pulsed magnetron sputtering is carried out as pulsed DC magnetron sputtering.

3. The process according to claim 1, **characterized in that** the pulsed magnetron sputtering is carried out as high-power impulse magnetron sputtering.

4. The process according to any of the preceding claims, **characterized in that** pulses having a peak power density of 0.01 kW/cm² to 30 kW/cm², in particular 0.01 kW/cm² to 0.5 kW/cm², preferably 0.01 kW/cm² to 0.1 kW/cm², are generated during the pulsed magnetron sputtering.

5. The process according to any of the preceding claims, **characterized in that** pulses having a frequency of 10 Hz to 300 kHz, in particular 10 kHz to 300 kHz, preferably 10 kHz to 100 kHz, are generated during the pulsed magnetron sputtering.

6. The process according to any of the preceding claims, **characterized in that** pulses having a voltage of 200 V to 800 V, preferably 300 V to 700 V, are generated during the pulsed magnetron sputtering.

7. The process according to any of the preceding claims, **characterized in that** pulses having a duration of 10 µs to 200 µs, preferably 40 µs to 150 µs, are generated during the pulsed magnetron sputtering.

8. The process according to any of the preceding claims, **characterized in that** a ceramic material selected from the group consisting of chromium nitride, chromium carbonitride, zirconium chromium nitride, zirconium nitride and combinations of at least two of the aforesaid ceramic materials is sputtered and deposited in layers on the surface of the implant during the pulsed magnetron sputtering to generate the ceramic multi-layer coating.

9. The process according to any of the preceding claims, **characterized in that** further a material, in particular a metallic material, preferably zirconium, or a metal-containing material, in particular metal nitride, preferably zirconium nitride, is sputtered in the presence of oxygen and/or nitrogen during the pulsed magnetron sputtering to form an outer oxide layer or an outer oxynitride layer, in particular outer metal oxide layer or outer metal oxynitride layer, of the ceramic multi-layer coating.

10. The process according to any of the preceding claims, **characterized in that** the following layers are deposited, in particular directly on top of each other, on the surface of the implant, preferably in the order a) to g) or optionally in the order a) to h), during the pulsed magnetron sputtering to generate the ceramic multi-layer coating:
a) a chromium nitride layer,
b) a chromium carbonitride layer,
c) a chromium nitride layer,
d) a chromium carbonitride layer,
e) a chromium nitride layer,
f) a zirconium chromium nitride layer,
g) a zirconium nitride layer and optionally
h) an oxide layer or an oxynitride layer, in particular a metal oxide layer, preferably zirconium oxide layer, or metal oxynitride layer, preferably zirconium oxynitride layer.

11. The process according to any of the preceding claims, **characterized in that** the surface of the implant is a concave surface and/or an articulating surface.

12. The process according to any of the preceding claims, **characterized in that** the implant is a joint implant, in particular selected from the group consisting of tibial implant, femoral implant, knee joint implant, hip joint implant, ankle joint implant, shoulder joint implant, mandibular joint implant, elbow joint implant, finger joint implant and spinal joint implant, or a component thereof.

13. An implant having a ceramic multi-layer coating, **characterized in that** the implant is produced or producible according to a process according to any of the preceding claims and/or that the ceramic multi-layer coating comprises an oxide layer or an oxynitride layer, in particular a metal oxide layer, preferably zirconium oxide layer, or metal oxynitride layer, preferably zirconium oxynitride layer, as an outer layer.

14. The implant according to claim 13, **characterized in that** the oxide layer or oxynitride layer, in particular metal oxide layer or metal oxynitride layer, in particular directly, layers a zirconium nitride layer of the ceramic multi-layer coating.

15. The implant according to claim 13 or 14, **characterized in that** the ceramic multi-layer coating comprises or consists of the following layers, preferably in the order a) to h):
a) a chromium nitride layer,
b) a chromium carbonitride layer,
c) a chromium nitride layer,
d) a chromium carbonitride layer,
e) a chromium nitride layer,
f) a zirconium chromium nitride layer,
g) a zirconium nitride layer and
h) the oxide layer or oxynitride layer, in particular metal oxide layer, preferably zirconium oxide layer, or metal oxynitride layer, preferably zirconium oxynitride layer.
